# EUROPÄISCHE PATENTSCHRIFT

(11) **EP 0 382 047 B1**
(45) Veröffentlichungstag und Bekanntmachung des Hinweises auf die Patenterteilung: **25.08.1993**
(21) Anmeldenummer: 90101772.3
(22) Anmeldetag: 30.01.1990
(51) Int. Cl.: A61L 27/00

(54) **Resorbierbare Knochenkeramik auf Basis von Tricalciumphosphat**
Tricalcium phosphate-based resorbable osseous ceramic
Céramique osseuse résorbable à base de phosphate tricalcique

(30) Priorität: 08.02.1989 DE 3903695
(43) Veröffentlichungstag der Anmeldung: 16.08.1990
(73) Patentinhaber: MERCK PATENT GmbH, D-64271 Darmstadt (DE)
(72) Erfinder: Bauer, Gerd, Dr., D-6100 Darmstadt 12 (DE)

(56) Entgegenhaltungen:
- EP-A- 0 141 004
- EP-A- 0 284 074
- EP-A- 0 303 941
- US-A- 4 314 380

## Beschreibung

Die Erfindung betrifft als Knochenersatzmaterial verwendbare resorbierbare Knochenkeramik auf Basis von Tricalciumphosphat und ein Verfahren zu dessen Herstellung.

An leistungsfähiges medizinisches Implantatmaterial wird heute die Forderung gestellt, daß es einerseits eine hohe mechanische Stabilität aufweist, andererseits aber auch eine hohe biologische Aktivität zeigt, nämlich dahingehend, daß es vom Organismus angenommen und in ihn integriert wird. Im Falle von Knochenersatzmaterial bedeutet dies, daß es bald mit körpereigenen Gewebe, insbesondere dem Knochen, verwachsen soll.

Implantatwerkstoffe auf mineralischer Basis gewährleisten meist nur dann eine hohe mechanische Stabilität, wenn sie als Keramiken, d. h. also in Form vom bei ausreichend hohen Temperaturen gesinterten Materialien bzw. Werkstücken, eingesetzt werden.

Es ist bekannt, daß aus natürlichem Knochenmaterial hergestelltes Knochenersatzmaterial vom Körpergewebe leichter angenommen und durchwachsen wird. Knochenkeramik besitzt eine hohe Bioaktivität, insbesondere dann, wenn hierin die poröse Feinstruktur, die für natürlichen Knochen charakteristisch ist, erhalten geblieben ist. Hierzu muß aus dem natürlichen Knochenmaterial der Anteil an organischen Substanzen entfernt werden, möglichst in eiener Weise, daß die mineralische Matrix unverändert bleibt. Diese muß anschließend zur Knochenkeramik gesintert werden.

Bei bekannten Verfahren wird ein wesentlicher Teil des organischen Materials nach der mechanischen Entfernung von Weichteilen durch chemische Behandlung aus dem Knochenmaterial herausgelöst, beispielsweise mit WasserstoffperoxidLösung. Abgesehen davon, daß derartige Behandlungen umständlich sind und beispielsweise wegen der bleichenden Wirkung von Wasserstoffperoxid Vorsichtsmaßnahmen erfordern, gelingt es auf diese Weise nicht vollständig, das organische Material aus dem Knochengerüst zu entfernen. Durch anschließend Pyrolyse des Knochens bei erhöhten Temperaturen läßt sich zwar das restliche organische Material verbrennen; dabei können aber flüchtige Bestandteile in solchen Mengen auftreten, daß dadurch die Feinstruktur der Knochenmatrix beschädigt wird. Dabei ist zu berücksichtigen, daß die Knochenmatrix ihre mechanische Festigkeit erst beim Sintern erhält; vor dem Sintern hingegen weist die Matrix nur eine sehr geringe mechanische Stabilität auf.

Für die Herstellung von Knochenkeramik unter vollständigem Erhalt der porösen Struktur natürlichen Knochens ist es daher erforderlich, daß die vollständige Entfernung organischer Substanzen unter besonders schonenden Bedingungen möglich ist.

Natürliches Knochenmaterial besteht in seiner Mineralphase praktische vollständig aus Hydroxylapatit (im folgenden mit HA abgekürzt), einem Calciumphosphat der Summenformel Cas(PO₄)aOH. Durch vorstehend skizzierte oder analoge Bearbeitungsmaßnahmen bis hin zur gebrannten Knochenkeramik wird die Mineralphase chemisch nicht verändert, liegt also im Endprodukt auch als HA vor.

Implantate und Knochenersatzmaterial auf Basis von HA gelten als im Organismus im wesentlichen nicht resorbierbar. Das heißt, das körperfremde Material bleibt über lange Zeit praktisch unverändert erhalten. Derartige Knochenkeramik hat aber eine geringere mechanische Festigkeit als natürlich gewachsener körpereigener Knochen.

Hierzu wären Materialien wesentlich günstiger, die nach Integration in das Körpergewebe und Verwachsung mit dem körpereigenen Knochen relativ rasch resorbiert und durch neugebildete körpereigene Knochensubstanz ersetzt werden.

Es ist bekannt, daß Materialien auf Basis von Tricalciumphosphat (im folgenden mit TCP abgekürzt) mit der Summenformel Ca₃(P0₄)₂ unter bestimmten Umständen eine hohe Resorbierbarkeit im Organismus zeigen. Hierzu muß das Material eine Hochtemperaturbehandlung durchlaufen haben, wie etwa bei der Herstellung gesinterter Keramik. Bei der Abkühlung tritt in TCP bei einer Temperatur von 1125 _{°}C ein Phasenwechsel in der Kristallmodifikation ein, der mit einer sprunghaften Volumenabnahme von über 7 % verbunden ist. Dies führt zwangsläufig zu erheblichen Spannungen und Mikrorissen im Materialgefüge. Dies hat zur Folge, daß im Organismus bald eine Degradation der Keramik zu feinen Körnern erfolgt, worauf das Material durch körperspezifische Lysierungsmechanismen resorbiert und damit dem Stoffwechsel und letztendlich auch dem Aufbau neuer körpereigener Knochensubstanz zugeführt wird.

Bekannte derartige Materialien auf Basis von TCP sind jedoch nicht aus natürlichem Knochen unter Erhalt der porösen Feinstruktur natürlichen Knochens hergestellt.

Der Erfindung liegt daher das besondere Bedürfnis nach einer Knochenkeramik zugrunde, die die Material- und Werkstoffeigenschaften von aus natürlichem Knochen unter Erhalt der porösen Struktur hergestellter, aber normalerweise aus HA bestehender Keramik mit der Resorbierbarkeit von TCP vereint.

Es wurde nun gefunden, daß sich eine derartige, als Knochernersatzmaterial verwendbare resorbierbare Knochenkeramik auf Basis von Tricalciumphosphat in der Weise herstellen läßt, daß man natürliches, von Weichteilen befreites Knochenmaterial als Ausgangsmaterial verwendet, aus diesem durch Pyrolyse restliche organische Substanzen entfernt, das verbleibende, aus Hydroxylapatit bestehende Knochenmaterial anschließend mit Phosphatträger behandelt und dann einer Sinterung unterzieht.

Das erfindungsgemäße Verfahren ist dadurch gekennzeichnet, daß man das Knochenmaterial nach der Entfernung der Weichteile bei einer erhöhten Temperatur bis maximal 150 °C trocknet, daran anschließend bei Luftunterschuß oder unter reduzierender Atmosphäre die Temperatur auf etwa 550 _{°} C erhöht, in einem nächsten Schritt das Knochenmaterial bei Luftüberschuß auf eine Temperatur zwischen 550_{°} und 800 °C bringt, nach der Abkühlung mit einer für eine Umwandlung in Tricalciumphosphat entsprechenden stöchiometrischen Menge an Phosphatträger behandelt und daran eine Sinterung bei Temperaturen zwischen 1125 und 1670 _{°} C anschließt.

Gegenstand der Erfindung ist auch eine resorbierbare Knochenkeramik, bei der es sich um ein aus natürlichem Knochenmaterial unter vollständigem Erhalt der porösen Feinstruktur, wie sie für natürlichen Knochen charakteristisch ist, hergestelltes, gesintertes Keramikmaterial handelt, das im wesentlichen aus Tricalciumphosphat besteht.

Gegenstand der Erfindung ist ein wie vorstehend charakterisiertes Verfahren zur Herstellung von resorbierbarer Knochenkeramik auf Basis von Tricalciumphosphat.

Das erfindungsgemäße Verfahren setzt sich aus zwei Hauptschritten zusammen, nämlich einer unter besonders schonenden Bedingungen ablaufenden Pyrolyse des organischen Anteils von natürlichem Knochenmaterial bei der die poröse Feinstruktur des Knochens vollständig erhalten bleibt, sowie einer gezielten Umwandlung der aus HA bestehenden Mineralphase des pyrolysierten Knochenmaterials in TCP durch Behandlung mit einem Phosphatträger und anschließende Sinterung.

Der erste Hauptschritt des erfindungsgemäßen Verfahrens wird in der Weise durchgeführt, daß man das Knochenmaterial nach der Entfernung der Weichteile bei einer erhöhten Temperatur bis maximal 150 °C trocknet, daran anschließend bei Luftunterschuß oder unter reduzierender Atmosphäre die Temperatur auf etwa 550 _{°} C erhöht und in einem nächsten Schritt das Knochenmaterial bei Luftüberschuß auf eine Temperatur zwischen 550 und 800 _{°} C bringt.

Um die Trocknung möglichst schonend durchzuführen, ist es vorteilhaft, den Knochen zwischen 12 und 72 Stunden bei einer erhöhten Temperatur von maximal 150 _{°} C zu trocknen. Außerdem ist es günstig, wenn man bei der Trocknung die Temperatur allmählich von Umgebungstemperatur auf die Maximaltemperatur von 150 _{°} C erhöht. Allmählich bedeutet dabei, daß die Erhöhung kontinuierlich über den gesamten Trocknungszeitraum erfolgt. Es wird dadurch vermieden, daß bei der Siedetemperatur des Wassers eine schlagartige Verdampfung einsetzt, die das Knochenmaterial beschädigen könnte. Durch die allmähliche Erhöhung der Temperatur erhält man ein stetiges Verdampfen der leicht flüchtigen Komponenten über den gesamten Trocknungszeitraum, wobei keine größeren Gasentwicklungen auftreten.

Dadurch, daß man nach der Trocknung das Knochenmaterial bei Luftunterschuß oder unter reduzierender Atmosphäre einer erhöhten Temperatur bis etwa 550 °C unterzieht, stellt sich eine Pyrolyse ein, also eine thermische Zersetzung des organischen Materials, welche zu einer Verkohlung des organischen Knochenmaterials unter Abspaltung flüchtiger Komponenten führt. Es ist hierbei vorteilhaft, wenn man den Knochen etwa 8-24 Stunden unter Luftunterschuß oder unter reduzierender Atmosphäre behandelt. Dabei wird die Temperatur vorzugsweise allmählich auf 550 _{°} C erhöht, wobei auch hier allmählich bedeutet, daß die Temperatur über den gesamten Behandlungszeitraum stetig erhöht wird. Auch diese Maßnahme trägt dazu bei, daß Gaskomponenten unterschiedlicher Flüchtigkeit nacheinander gebildet werden, so daß zu keinem Zeitpunkt unerwünscht hohe Gasentwicklungen auftreten. Im nächsten Schritt wird dann bei Luftüberschuß bei Temperaturen bis maximal 800 _{°} C die Kohle verbrannt. Leicht flüchtige Komponenten sind bei diesem reinen Verbrennungsvorgang nicht mehr vorhanden, so daß eine Beschädigung der anorganischen Knochenmatrix vermieden wird.

Das Brennen des Knochenmaterials bei Luftüberschuß bei einer Temperatur von 750 bis 800 °C wird vorzugsweise 1 bis 24 Stunden lang durchgeführt, wobei es auch hier günstig ist, bei dem Brennen des Knochens die Temperatur allmählich auf etwa 800 _{°} C zu steigern.

Das beschriebene Verfahren der schonenden Pyrolyse hat den Vorteil, daß keinerlei chemische Behandlung des Knochenmaterials notwendig ist, sondern daß nach der mechanischen Entfernung der Weichteile das Knochenmaterial ausschließlich einer Wärmebehandlung unterzogen wird, die in verschiedene Abschnitte unterteilt ist. Die so erhaltene mineralische Knochenmatrix hat die unveränderte Feinstruktur des eingesetzten Knochenmaterials und besteht unverändert aus HA.

In dem zweiten Hauptschritt des erfindungsgemäßen Verfahrens wird die chemische Zusammensetzung der Mineralphase des pyrolysierten Knochenmaterials von HA in TCP umgewandelt. Hierzu wird das Material mit einer stöchiometrisch entsprechenden Menge eines Phosphatträgers behandelt und anschließend einer Sinterung unterzogen.

Eine vom Prinzip her vergleichbare Umwandlung von HA natürlichen Ursprungs in TCP und/oder andere Calciumphosphate ist aus EP 278 583 bekannt. Dort ist die Herstellung von porösem Knochenersatzmaterial beschrieben, bei dem die mineralische Matrix matinen Ursprungs (Korallen) ist. Das dort beschriebene Verfahren ist dahingehend nachteilig, daß es eine zweimalige chemische Umwandlung des Materials beinhaltet, nämlich zunächst eine hydrothermale Umwandlung des natürlichen Calicumcarbonatmaterials in HA und daran anschließend die Umwandlung in TCP und/oder andere Calciumphosphate durch Behandlung mit Phosphatträger und Sinterung. Eine gezielte stöchiometrische Umsetzung zu praktisch ausschließlich TCP-Keramik ist mit dem beschriebenen Verfahren nicht möglich oder zumindest nicht vorgesehen. Ein weiterer gravierender Nachteil ist, daß dieses Material aufgrund des marinen Ursprungs seines Ausgangsmaterials nicht die für natürlichen Knochen typische Feinstruktur und auch nicht die Spurenelementverteilung natürlichen Knochens besitzen kann.

Gemäß der Erfindung erfolgt die Umwandlung der aus HA bestehenden Mineralphase des pyrolysierten Knochenmaterials in TCP durch Behandlung mit einem Phosphatträger und Sinterung. Als Phosphatträger sind im Prinzip alle phosphathaltigen Verbindungen geeignet, die sich thermisch so zersetzen lassen, daß sie praktisch nur ihren Gehalt an P₂0₅ an das HA-Material abgeben. Derartige Verbindungen sind vorzugsweise Phosphorsäure, Ammoniumdihydrogenphosphat. Diammoniumhydrogenphosphat oder Triammoniumphosphat. Diese Verbindungen spalten bei erhöhter Temperatur Wasser bzw. Wasser und Ammoniak ab; der Anteil an P₂0₅ verbleibt im wesentlichen in der Mineralphase, wobei deren Stöchiometrie von HA nach TCP verschoben wird. Als ein besonders günstiger und daher bevorzugter Phosphatträger hat sich ein phosphorreiches Calciumphosphat der stöchiometrischen Zusammensetzung CaO . 2 P₂0₅ erwiesen, mit dessen Einsatz Sublimationsverluste von P₂0₅ praktisch ausgeschlossen sind, wodurch das Endprodukt aus einer praktisch vollständig in TCP umgewandelten Mineralphase besteht.

Das abgekühlte pyrolysierte Knochenmaterial wird mit dem Phosphatträger bevorzugt in Form wäßriger Lösung behandelt, wobei sich die erforderlichen Mengen und Konzentrationen im jeweiligen Fall durch einfache stöchiometrische Berechnungen festlegen lassen. Die Behandlung erfolgt durch Tränken und Trocknen. Danach wird die Hochtemperaturbehandlung bei Temperaturen zwischen 1125 und 1670 °C durchgeführt, wobei die Zersetzung des Phosphatträgers, die Umwandlung von HA in TCP und die Sinterung zur Keramik erfolgt. Die Sinterung wird vorzugsweise über einen Zeitraum von 2-6 Stunden und bei Temperaturen zwischen 1150 und 1400 ° C durchgeführt. Auch hierbei ist es vorteilhaft, die Temperatur allmählich auf die Sintertemperatur zu erhöhen. Danach erfolgt die Abkühlung, vorzugswiese langsam über einen Zeitraum von 2-6 Stunden hinweg.

Die so erhaltene Knochenkeramik hat die Werkstoffeigenschaften, insbesondere mechanische Festigkeit, wie sie für hochgebrannte, gesinterte Keramikmaterialien typisch ist. Da sie erfindungsgemäß unter praktisch vollständigem Erhalt der natürlichen porösen Feinstruktur des Knochen-Ausgangsmaterials hergestellt ist, verfügt sie über eine hohe Bioaktivität, die sich in einer schnellen und intensiven Durchwachsung und Verwachsung mit körpereigenem Gewebe zeigt. Da sie im wesentlichen aus TCP besteht, ist sie darüberhinaus resorbierbar, so daß sie nach Einsatz in den Organismus bald durch neugebildete körpereigene Knochensubstanz ersetzt wird.

### Beispiel 1

Rinderknochen wurde durch Auskochen teilweise von seinen organischen Bestandteilen befreit. Anhaftende Weichteile wurden mechanisch entfernt. Die gewünschte Formgebung erfolgte durch Zerkleinerung mittels Säge.

Danach wurde die Trocknung mit kontinierlicher Temperatursteigerung von Raumtemperatur bis 150 °C im Verlauf von 26 h durchgeführt. Es erfolgte die Pyrolyse der organischen Bestandteile durch Temperatursteigerung mit 25 _{°}C/h unter Luftunterschuß auf 550 _{°}C. Danach wurde das Material mit 25 °C/h im Luftüberschuß auf 800 ° C gebracht.

Nach der Abkühlung wurde mit 33 Gew.% einer wäßrigen Lösung von 40,8 % (NH₄)₂HPO₄ getränkt und getrocknet. Die Sinterung erfolgte bei 1250 _{°} C über 4 h nach einer Aufheizung mit 100 °C/h.

Die erhaltene Knochenkeramik besitzt röntgenographisch einen TCP-Gehalt von über 95 %.

### Beispiel 2

Rinderknochen wurde vorbereitet und pyrolysiert wie in Beispiel 1.

Nach der Abkühlung wurde mit 33 Gew.% einer wäßrigen Lösung die 200 g CaO . 2 P₂0₅ -(hergestellt aus Ca(H2P04)2 . H₂0 und H₃P0₄ im Molverhältnis 1:2) pro Liter enthält, getränkt und getrocknet.

Die Sinterung erfolgte bei 1250 °C über 4 h nach einer Aufheizung mit 100 _{°} C/h.

Die erhaltene Knochenkeramik besitzt röntgenographisch einen TCP-Gehalt von über 95 %.

## Patentansprüche (Patentansprüche für folgende(n) Vertragsstaat(en) : AT, BE, CH, DE, FR, GB, IT, LI, LU, NL, SE)

1. Resorbierbare Knochenkeramik auf Basis von Tricalciumphosphat, dadurch gekennzeichnet, daß es sich um ein aus natürlichem Knochenmaterial unter vollständigem Erhalt der porösen Feinstruktur, wie sie für natürlichen Knochen charakteristisch ist, hergestelltes, gesintertes Keramikmaterial handelt, das im wesentlichen aus Tricalciumphosphat besteht.

2. Verfahren zur Herstellung von als Knochenersatzmaterial verwendbarer resorbierbarer Knochenkeramik auf Basis von Tricalciumphosphat, dadurch gekennzeichnet, daß man natürliches, von Weichteilen befreites Knochenmaterial als Ausgangsmaterial verwendet, wobei man das Knochenmaterial nach der Entfernung der Weichteile bei einer erhöhten Temperatur bis maximal 150 _{°} C trocknet, daran anschließend bei Luftunterschuß oder unter reduzierender Atmosphäre die Temperatur auf etwa 550 _{°} C erhöht, in einem nächsten Schritt das Knochenmaterial bei Luftüberschuß auf eine Temperatur zwischen 550 _{°} C und 800 _{°} C bringt, nach der Abkühlung mit einer für eine Umwandlung in Tricalciumphosphat entsprechenden stöchiometrischen Menge an Phosphatträger behandelt und daran eine Sinterung bei Temperaturen zwischen 1125 und 1670 °C anschließt.

3. Verfahren nach Anspruch 2, dadurch gekennzeichnet, daß man das Knochenmaterial zwischen 12 und 72 Stunden bei einer erhöhten Temperatur von maximal 150 _{°} C trocknet.

4. Verfahren nach Anspruch 3, dadurch gekennzeichnet, daß man bei der Trocknung die Temperatur allmählich von Umgebungstemperatur auf die Maximaltemperatur 150 °C erhöht.

5. Verfahren nach mindestens einem der Ansprüche 3 bis 4, dadurch gekennzeichnet, daß man nach der Trocknung das Knochenmaterial etwa 8 bis 24 Stunden unter Luftunterschuß oder unter reduzierender Atmosphäre bei einer Temperatur bis etwa 550 _{°} C behandelt.

6. Verfahren nach Anspruch 5, dadurch gekennzeichnet, daß man die Temperatur während der Behandlung des Knochenmaterials unter Luftunterschuß oder einer reduzierenden Atmosphäre allmählich auf 550 °C erhöht.

7. Verfahren nach mindestens einem der Ansprüche 2 bis 6, dadurch gekennzeichnet, daß man für die Pyrolyse das Knochenmaterial etwa 1 bis 24 Stunden bei Luftüberschuß auf einer Temperatur zwischen 550 °C und 800 °C hält.

8. Verfahren nach Anspruch 7, dadurch gekennzeichnet, daß man bei der Pyrolyse des Knochenmaterials unter Luftüberschuß die Temperatur allmählich bis auf etwa 800 _{°} C steigert.

9. Verfahren nach mindestens einem der Ansprüche 2 bis 8, dadurch gekennzeichnet, daß man nach erfolgter Pyrolyse und Abkühlung mit einer wäßrigen Lösung eines Phosphatträgers versetzt und trocknet.

10. Verfahren nach Anspruch 9, dadurch gekennzeichnet, daß man als Phosphatträger Phosphorsäure, Ammoniumdihydrogenphosphat, Diammoniumhydrogenphosphat oder Triammoniumphosphat einsetzt.

11. Verfahren nach Anspruch 9, dadurch gekennzeichnet, daß man als Phosphatträger ein phosphorreiches Calciumphosphat der stöchiometrischen Zusammensetzung CaO . 2 P₂0₅ einsetzt.

12. Verfahren nach mindestens einem der Ansprüche 2 bis 10, dadurch gekennzeichnet, daß die Sinterung über einen Zeitraum von 2 bis 6 Stunden und bei einer Temperatur zwischen 1150 und 1400 °C erfolgt.

13. Verfahren nach Anspruch 12, dadurch gekennzeichnet, daß man die Temperatur allmählich auf die Sintertemperatur erhöht.

## Patentansprüche (Patentansprüche für folgende(n) Vertragsstaat(en) : ES, GR)

1. Verfahren zur Herstellung von als Knochenersatzmaterial verwendbarer resorbierbarer Knochenkeramik auf Basis von Tricalcium phosphat, dadurch gekennzeichnet, daß man natürliches, von Weichteilen befreites knochenmaterial als Ausgangsmaterial verwendet, wobei man das Knochenmaterial nach der Entfernung der Weichteile bei einer erhöhten Temperatur bis maximal 150 °C trocknet, daran anschließend bei Luftunterschuß oder unter reduzierender Atmosphäre die Temperatur auf etwa 550 °C erhöht, in einem nächsten Schritt das Knochenmaterial bei Luftüberschuß auf eine Temperatur zwischen 550 °C und 800 °C bringt, nach der Abkühlung mit einer für eine Umwandlung in Tricalciumphosphat entsprechenden stöchiometrischen Menge an Phosphatträger behandelt und daran eine Sinterung bei Temperaturen zwischen 1125 und 1670 _{°} C anschließt.

2. Verfahren nach Anspruch 1, dadurch gekennzeichnet, daß man das Knochenmaterial zwischen 12 und 72 Stunden bei einer erhöhten Temperatur von maximal 150 _{°} C trocknet.

3. Verfahren nach Anspruch 2 , dadurch gekennzeichnet, daß man bei der Trocknung die Temperatur allmählich von Umgebungstemperatur auf die Maximaltemperatur 150 _{°} C erhöht.

4. Verfahren nach mindestens einem der Ansprüche 2 bis 3, dadurch gekennzeichnet, daß man nach der Trocknung das Knochenmaterial etwa 8 bis 24 Stunden unter Luftunterschuß oder unter reduzierender Atmosphäre bei einer Temperatur bis etwa 550 _{°} C behandelt.

5. Verfahren nach Anspruch 4, dadurch gekennzeichnet, daß man die Temperatur während der Behandlung des Knochenmaterials unter Luftunterschuß oder einer reduzierenden Atmosphäre allmählich auf 550 °C erhöht.

6. Verfahren nach mindestens einem der Ansprüche 1 bis 5, dadurch gekennzeichnet, daß man für die Pyrolyse das Knochenmaterial etwa 1 bis 24 Stunden bei Luftüberschuß auf einer Temperatur zwischen 550 °C und 800 °C hält.

7. Verfahren nach Anspruch 6, dadurch gekennzeichnet, daß man bei der Pyrolyse des Knochenmaterials unter Luftüberschuß die Temperatur allmählich bis auf etwa 800 _{°} C steigert.

8. Verfahren nach mindestens einem der Ansprüche 1 bis 7, dadurch gekennzeichnet, daß man nach erfolgter Pyrolyse und Abkühlung mit einer wäßrigen Lösung eines Phosphatträgers versetzt und trocknet.

9. Verfahren nach Anspruch 8, dadurch gekennzeichnet, daß man als Phosphatträger Phosphorsäure, Ammoniumdihydrogenphosphat, Diammoniumhydrogenphosphat oder Triammoniumphosphat einsetzt.

10. Verfahren nach Anspruch 8, dadurch gekennzeichnet, daß man als Phosphatträger ein phosphorreiches Calciumphosphat der stöchiometrischen Zusammensetzung CaO . 2 P₂0₅ einsetzt.

11. Verfahren nach mindestens einem der Ansprüche 1 bis 9, dadurch gekennzeichnet, daß die Sinterung über einen Zeitraum von 2 bis 6 Stunden und bei einer Temperatur zwischen 1150 und 1400 °C erfolgt.

12. Verfahren nach Anspruch 11, dadurch gekennzeichnet, daß man die Temperatur allmählich auf die Sintertemperatur erhöht.

## Claims (Claims for the following Contracting State(s) : AT, BE, CH, DE, FR, GB, IT, LI, LU, NL, SE)

1. Resorptive bone ceramic on the basis of tricalcium phosphate, characterized in that it is a sintered ceramic material which essentially consists of tricalcium phosphate and which is made of natural bone material while the porous fine structure, as is characteristic of natural bone, remains completely intact.

2. Process for the preparation of resorptive bone ceramic on the basis of tricalcium phosphate, which can be used as a bone replacement material, characterised in that natural bone material which has been freed from soft tissues is used as the starting material, where, the soft tissues having first been removed, the bone material is dried at an increased temperature of not more than 150°C, the temperature is then increased to about 550°C in a reducing atmosphere or an atmosphere deficient in air, the bone material is in a next step brought to a temperature of between 550 ° and 800 _{°} C under an excess of air, cooled, then treated with such a stoichiometric amount of phosphate carrier as is required for converting it into tricalcium phosphate, followed by a sintering step at temperatures of between 1125 and 1670°C.

3. Process according to Claim 2, characterized in that the bone material is dried for between 12 and 72 hours at an increased temperature of not more than 150°C.

4. Process according to Claim 3, characterized in that, during the drying process, the temperature is gradually increased from ambient temperature to the maximum temperature of 150_{°}C.

5. Process according to at least one of Claims 3 to 4, characterized in that, after the drying process, the bone material is treated for about 8 to 24 hours at a temperature of up to about 550°C under a reducing atmosphere or an atmosphere deficient in air.

6. Process according to Claim 5, characterized in that the temperature during the treatment of the bone material is gradually increased to 550°C in a reducing atmosphere or an atmosphere deficient in air.

7. Process according to at least one of Claims 2 to 6, characterized in that, for the pyrolysis step, the bone material is kept for about 1 to 24 hours at a temperature of between 550 °C and 800 _{°} C in an excess of air.

8. Process according to Claim 7, characterized in that, during pyrolysis of the bone material, the temperature is gradually increased up to about 800 _{°} C in an excess of air.

9. Process according to at least one of Claims 2 to 8, characterized in that, when pyrolysis and cooling have been effected, the material is treated with an aqueous solution of a phosphate carrier and dried.

10. Process according to Claim 9, characterized in that phosphoric acid, ammonium dihydrogen phosphate, diammonium hydrogen phosphate or triammonium phosphate are employed as the phosphate carrier.

11. Process according to Claim 9, characterized in that a phosphorus-rich calcium phosphate of the stoichiometric composition CaO * 2 P₂0₅ is employed as the phosphate carrier.

12. Process according to at least one of Claims 2 to 10, characterized in that sintering is effected over a period of 2 to 6 hours and at a temperature of between 1150 and 1400 _{°} C.

13. Process according to Claim 12, characterized in that the temperature is gradually increased to the sintering temperature.

## Claims (Claims for the following Contracting State(s) : ES, GR)

1. Process for the preparation of resorptive bone ceramic on the basis of tricalcium phosphate, which can be used as a bone replacement material, characterised in that natural bone material which has been freed from soft tissues is used as the starting material, where, the soft tissues having first been removed, the bone material is dried at an increased temperature of not more than 150°C, the temperature is then increased to about 550 °C in a reducing atmosphere or an atmosphere deficient in air, the bone material is in a next step brought to a temperature of between 550 ° and 800 _{°} C under an excess of air, cooled, then treated with such a stoichiometric amount of phosphate carrier as is required for converting it into tricalcium phosphate, followed by a sintering step at temperatures of between 1125 and 1670°C.

2. Process according to Claim 1, characterized in that the bone material is dried for between 12 and 72 hours at an increased temperature of not more than 150°C.

3. Process according to Claim 2, characterized in that, during the drying process, the temperature is gradually increased from ambient temperature to the maximum temperature of 150_{°}C.

4. Process according to at least one of Claims 2 to 3, characterized in that, after the drying process, the bone material is treated for about 8 to 24 hours at a temperature of up to about 550°C under a reducing atmosphere or an atmosphere deficient in air.

5. Process according to Claim 4, characterized in that the temperature during the treatment of the bone material is gradually increased to 550°C in a reducing atmosphere or an atmosphere deficient in air.

6. Process according to at least one of Claims 1 to 5, characterized in that, for the pyrolysis step, the bone material is kept for about 1 to 24 hours at a temperature of between 550°C and 800 _{°} C in an excess of air.

7. Process according to Claim 6, characterized in that, during pyrolysis of the bone material, the temperature is gradually increased up to about 800 _{°} C in an excess of air.

8. Process according to at least one of Claims 1 to 7, characterized in that, when pyrolysis and cooling have been effected, the material is treated with an aqueous solution of a phosphate carrier and dried.

9. Process according to Claim 8, characterized in that phosphoric acid, ammonium dihydrogen phosphate, diammonium hydrogen phosphate or triammonium phosphate are employed as the phosphate carrier.

10. Process according to Claim 8, characterized in that a phosphorus-rich calcium phosphate of the stoichiometric composition CaO * 2 P₂0₅ is employed as the phosphate carrier.

11. Process according to at least one of Claims 1 to 9, characterized in that sintering is effected over a period of 2 to 6 hours and at a temperature of between 1150 and 1400 _{°} C.

12. Process according to Claim 11, characterized in that the temperature is gradually increased to the sintering temperature.

## Revendications (Revendications pour l'(les) Etat(s) contractant(s) suivant(s) : suivants : AT, BE, CH, DE FR, GB, IT, LI, LU, NL, SE)

1. Céramique d'os résorbable à base de phosphate tricalcique, caractérisée en ce qu'elle consiste en une matière céramique frittée préparée à partir de matière d'os naturelle avec conservation complète de la structure poreuse fine caractéristique des os naturels, et en ce qu'elle est composée essentiellement de phosphate tricalcique.

2. Procédé pour la préparation d'une céramique d'os résorbable à base de phosphate tricalcique utilisable en tant que matière de remplacement des os, caractérisé en ce que l'on utilise en tant que matière première de la matière d'os naturelle débarrassée des parties molles, qu'on sèche après élimination des parties molles à une température allant jusqu'à 150°C au maximum, après quoi on porte la température à 550 _{°} C environ sous défaut d'air ou en atmosphère réductrice, on porte la matière d'os dans le stade suivant à une température de 550 à 800 °C dans un excès d'air, on la traite après refroidissement par une matière phosphatée en quantité stoechiométrique pour une conversion en phosphate tricalcique puis on procède à un frittage à des températures de 1125 à 1670 °C.

3. Procédé selon la revendication 2, caractérisé en ce que la matière d'os est séchée en une durée de 12 à 72 heures à une température maximale de 150°C.

4. Procédé selon la revendication 3, caractérisé en ce que, pour le séchage, on porte la température peu à peu de la température ambiante jusqu'à la température maximale de 150°C.

5. Procédé selon au moins l'une des revendications 3 à 4, caractérisé en ce que, après le séchage, on traite la matière d'os pendant une durée d'environ 8 à 24 heures sous défaut d'air ou en atmosphère réductrice à une température allant jusqu'à 550 °C environ.

6. Procédé selon la revendication 5, caractérisé en ce que, durant le traitement de la matière d'os sous défaut d'air ou en atmosphère réductrice, on porte peu à peu la température à 5500 C.

7. Procédé selon au moins l'une des revendications 2 à 6, caractérisé en ce que, pour la pyrolyse, on maintient la matière d'os pendant une durée d'environ 1 à 24 heures sous excès d'air à une température de 550 à 800 _{°} C.

8. Procédé selon la revendication 7, caractérisé en ce que, à la pyrolyse de la matière d'os sous excès d'air, on porte la température peu à peu à un niveau d'environ 800 _{°} C.

9. Procédé selon au moins l'une des revendications 2 à 8, caractérisé en ce que, après pyrolyse et refroidissement, on ajoute une solution aqueuse d'une matière phosphatée et on sèche.

10. Procédé selon la revendication 9, caractérisé en ce que l'on utilise en tant que matière phosphatée de l'acide phosphorique, du phosphate monoammonique, du phosphate diam- monique ou du phosphate triammonique.

11. Procédé selon la revendication 9, caractérisé en ce que l'on utilise en tant que matière phosphatée un phosphate de calcium riche en phosphore, de composition stoechiométrique CaO, 2 P₂O₅.

12. Procédé selon au moins l'une des revendications 2 à 10, caractérisé en ce que l'on fritte pendant une durée de 2 à 6 heures à une température de 1150 à 1450°C.

13. Procédé selon la revendication 12, caractérisé en ce que l'on porte la température peu à peu à la température de frittage.

## Revendications (Revendications pour l'(les) Etat(s) contractant(s) suivant(s) : suivants : ES, GR)

1. Procédé de préparation d'une céramique d'os résorbable, à base de phosphate tricalcique, utilisable en tant que matière de remplacement des os, caractérisé en ce que l'on utilise en tant que matière première une matière d'os naturelle débarrassée des parties molles, on sèche la matière d'os, après élimination des parties molles, à une température de 150_{°}C au maximum, on porte ensuite la température à 550 °C environ sous défaut d'air ou en atmosphère réductrice, on porte la matière d'os, dans l'opération suivante, à une température de 550°C à 800°C sous excès d'air, on la traite après refroidissement par une matière phosphatée en quantité stoechiométrique pour une conversion en phosphate tricalcique puis on fait suivre d'un frittage à des températures de 1125 à 1670 °C.

2. Procédé selon la revendication 1, caractérisé en ce que l'on sèche la matière d'os pendant une durée de 12 à 72 heures à une température de 150°C au maximum.

3. Procédé selon la revendication 2, caractérisé en ce que, au séchage, on porte peu à peu la température depuis la température ambiante jusqu'à la température maximale de 150°C.

4. Procédé selon au moins l'une des revendications 2 à 3, caractérisé en ce que, après le séchage, on traite la matière d'os pendant une durée d'environ 8 à 24 heures sous défaut d'air ou en atmosphère réductrice à une température allant jusqu'à 550 °C environ.

5. Procédé selon la revendication 4, caractérisé en ce que, durant le traitement de la matière d'os sous défaut d'air ou en atmosphère réductrice, on porte peu à peu la température à 550 _{°} C.

6. Procédé selon au moins l'une des revendications 1 à 5, caractérisé en ce que, pour la pyrolyse de la matière d'os, on maintient celle-ci pendant environ 1 à 24 heures sous excès d'air à une température de 550 à 800 _{°} C.

7. Procédé selon la revendication 6, caractérisé en ce que, à la pyrolyse de la matière d'os sous excès d'air, on porte peu à peu la température de 800 °C environ.

8. Procédé selon au moins l'une des revendications 1 à 7, caractérisé en ce que, après pyrolyse et refroidissement, on ajoute une solution aqueuse d'une matière phosphatée et on sèche.

9. Procédé selon la revendication 8, caractérisé en ce que l'on utilise en tant que matière phosphatée de l'acide phosphorique, du phosphate monoammonique, du phosphate diam- monique ou du phosphate triammonique.

10. Procédé selon la revendication 8, caractérisé en ce que l'on utilise en tant que matière phosphatée un phosphate de calcium riche en phosphore de composition stoechiométrique CaO, 2 P₂O₅ .

11. Procédé selon au moins l'une des revendications 1 à 9, caractérisé en ce que le frittage est réalisé en une durée de 2 à 6 heures à une température de 1150 à 1400°C.

12. Procédé selon la revendication 11, caractérisé en ce que l'on porte peu à peu la température à la température de frittage.
